# EUROPÄISCHE PATENTSCHRIFT

(11) **EP 2 815 213 B1**
(45) Veröffentlichungstag und Bekanntmachung des Hinweises auf die Patenterteilung: **18.04.2018**
(21) Anmeldenummer: 13707104.9
(22) Anmeldetag: 15.02.2013
(51) Int. Cl.: G01D 5/241, A61B 5/00, G01L 1/14

(54) **TEXTILER DRUCKSENSOR**
TEXTILE PRESSURE SENSOR
DÉTECTEUR DE PRESSION TEXTILE

(30) Priorität: 16.02.2012 DE 102012101250; 03.05.2012 DE 102012103856
(43) Veröffentlichungstag der Anmeldung: 24.12.2014
(73) Patentinhaber: Seitz, Peter, D-81675 München (DE)
(72) Erfinder: Seitz, Peter, D-81675 München (DE)
(74) Vertreter: Bohnenberger, Johannes
(86) Internationale Anmeldenummer: PCT/EP2013/000455
(87) Internationale Veröffentlichungsnummer: WO 2013/120624

(56) Entgegenhaltungen:
- EP-A2- 0 172 784
- US-A1- 2002 121 146

## Beschreibung

Die Erfindung betrifft einen textilen Drucksensor zur kapazitiven Messung einer Druckverteilung auf einer Fläche gemäß dem Oberbegriff des Anspruchs 1 und ein Herstellungsverfahren nach dem Oberbegriff des Anspruchs 10.

Ein textiler Drucksensor zur kapazitiven Messung der Druckverteilung auf einer Fläche ist beispielsweise aus der DE 37 04 870 bekannt und zeigt eine Matrixanordnung von gegeneinander isolierten Streifen aus leitender Folie. Die Anordnung ist flexibel aber nicht dehnbar.

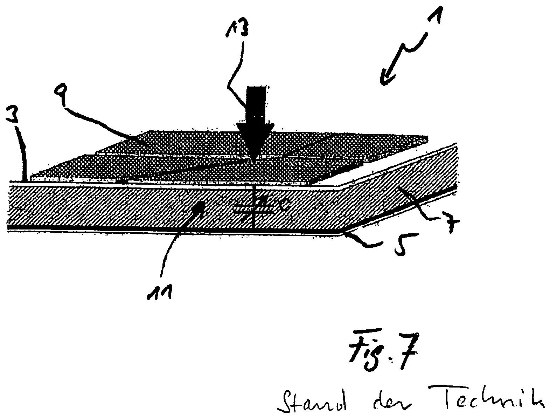

Ein aus der WO 2005/121729 bekannter gattungsgemäßer Drucksensor 1 zur kapazitiven Messung einer Druckverteilung auf einer Fläche ist in der beiliegenden Figur 7 gezeigt. Der Drucksensor 1 ist mattenförmig, d.h. im Wesentlichen flach ausgebildet und umfasst eine erste leitfähige Struktur 3 sowie eine zweite leitfähige Struktur 5. Zwischen der ersten und der zweiten leitfähigen Struktur, die ein Gewebe umfassen, ist ein dielektrisches Zwischenelement 7 angeordnet, welches reversibel komprimierbar, beispielsweise aus Filz oder Schaumstoff, ausgebildet ist. Die erste und die zweite Struktur sind also einander gegenüberliegend angeordnet. Die erste Struktur 3, die in der Figur 7 die Oberseite des textilen Drucksensors 1 bildet, umfasst mehrere leitfähige Bereiche 9, d.h. Elektroden, die voneinander elektrisch isoliert sind. Jeder leitfähige Bereich 9 ist jeweils über eine in der Figur 7 nicht gezeigte Verbindungsleitung mit einer elektrischen Schaltung, zum Zwecke der Energieversorgung und der Auswertung, verbunden. Als Oberseite des textilen Drucksensors 1 wird dabei diejenige Fläche oder Seite bezeichnet, die mit einem Objekt, dessen Druckverteilung auf dem textilen Drucksensor 1 gemessen werden soll, in Kontakt kommt. In der gezeigten Ausführungsform weist die zweite Struktur 5 eine durchgehend leitfähige Fläche auf, jedoch ist es ebenso denkbar, mehrere von einander isolierte leitfähige Bereiche entsprechend der ersten Struktur 3 vorzusehen. Die zweite Struktur 5 bildet die Unterseite des textilen Drucksensors 1, die im Wesentlichen parallel zu der Oberseite des Drucksensors angeordnet ist. Durch die gegenüberliegende Anordnung von leitfähigen Bereichen werden Kondensatoren C zwischen der ersten Struktur 3 und der zweiten Struktur 5 in Überschneidungsbereichen 11 ausgebildet.

Aus der EP 1 927 825 A1 ist ein kapazitiver Sensor bekannt, der als Einzelsensor aufgebaut ist. Hierbei kommen Gewebe zum Einsatz, auf welche Folien als Kondensatorplatten appliziert werden. Die Gewebe können auch als Gestricke ausgebildet sein.

Aus der US 2007/0248799 A1 ist ein ähnlicher Aufbau bekannt, wobei leitende oder nichtleitende Gewebe zum Einsatz kommen. Auch hier ist unter dem Begriff "Gewebe" auch Maschenware, also Gestricke oder Gewirke zu verstehen. Auch hier ist der bekannte Aufbau sehr arbeitsaufwändig.

Weiterhin sind aus der US 2002/121146 A1 und der EP 0 172 784 A2 kapazitive Sensoren bekannt, bei denen Gewebe aus nicht leitenden Kettfäden und leitenden Schussfäden zur Herstellung der Elektroden vorgeschlagen werden. Die Herstellung von größeren Flächen von Sensoren ist unter Verwendung dieser bekannten Techniken sehr aufwändig.

Die Funktionsweise von kapazitiven Sensoren ist hinlänglich bekannt. Sie arbeiten auf Basis der Veränderung der Kapazität eines einzelnen Kondensators oder eines ganzen Kondensatorsystems. Fast alle kapazitiven Sensoren basieren auf dem Prinzip, dass zwei Platten einen elektrischen Kondensator bilden, von denen eine Platte durch den zu messenden Effekt verschoben oder verformt wird. Dadurch ändert sich der Plattenabstand und damit die elektrisch messbare Kapazität. Um auch kleine Veränderungen besser detektieren zu können ist die eigentliche Messelektrode häufig mit einer Schirmelektrode umgeben, die den inhomogenen Randbereich des elektrischen Felds von der Messelektrode abschirmt. Dadurch ergibt sich zwischen Messelektrode und üblicherweise geerdeter Gegenelektrode ein annähernd paralleles elektrisches Feld mit der bekannten Charakteristik eines idealen Plattenkondensators. Bei einem kapazitiven Drucksensor wird die Kapazitätsänderung infolge des Durchbiegens einer Membrane und der resultierenden Änderung des Plattenabstandes als Sensoreffekt ausgewertet.

Die Membran wird hierbei als Kondensatorplatte ausgebildet. Die Kapazitätsänderungen sind ziemlich klein, so dass eine geeignete Verarbeitungselektronik mit hoher Empfindlichkeit integriert sein muss.

Bezogen auf den textilen Drucksensor 1 bedeutet dies, dass zur Messung einer Druckverteilung ein Objekt mit der Oberseite des textilen Drucksensors 1 in Kontakt kommt. Das Objekt übt dabei eine Druckkraft in Richtung des Pfeils 13 auf den textilen Drucksensor 1 aus, wodurch eine Kompression der dielektrischen Zwischenschicht 7 bewirkt wird. Mit anderen Worten reduziert sich der Abstand zwischen den leitfähigen Bereichen 9 der Strukturen 3 und 5 dort, wo die Kraft durch das Objekt auf die Oberseite des textilen Drucksensors 1 ausgeübt wird.

Die Kompression der Zwischenschicht 7 und damit eine Verringerung des Abstands zwischen den Strukturen 3 und 5 ist dort am größten, wo die größte Druckkraft auf die Oberseite des textilen Drucksensors 1 ausgeübt wird. Entsprechend wird in den Breichen der größten Krafteinwirkung bzw. des größten Drucks die Kapazitätsänderung am größten sein, die von einer angeschlossenen Auswertelektronik erfasst wird. Die leitfähigen Bereiche 9 sind vorzugsweise matrixförmig angeordnet und erzeugen somit eine matrixförmige Anordnung von Kondensatoren C, welche vorzugsweise die gesamte Fläche des textilen Drucksensors 1 überspannen. Durch die Erfassung der Kapazität und insbesondere der Kapazitätsänderung eines jeden Kondensators C kann somit ein Druckverteilungsmuster durch den textilen Drucksensor 1 erzeugt werden.

Die Anwendung von textilen Drucksensoren der hier angesprochenen Art ist nicht auf einen bestimmten Bereich beschränkt. Vielmehr sind sie vielfältig einsetzbar, beispielsweise im Bereich der Medizintechnik, Orthopädie oder Sport. Beispielsweise können textile Drucksensoren in der Medizintechnik zur Vermeidung von Dekubitalgeschwüren zum Einsatz kommen, d.h. von lokalen Schädigungen der Haut und des darunterliegenden Gewebes durch Druckausübung auf Körperteile eines Patienten. Derartige Dekubitalgeschwüre können beispielsweise bei bettlägerigen oder an den Rollstuhl gebundenen Patienten auftreten. Textile Drucksensoren können dazu eingesetzt werden, die flächige Druckverteilung eines Körperteils auf einer Unterlage, beispielsweise auf einer Matratze oder dergleichen, zu messen und Druckstellen zu identifizieren. Im Bereich der Medizintechnik kann ferner die Druckverteilung beim Gehen eines Probanden beispielsweise Aufschluss über orthopädische Schädigungen oder auch sensorische Schädigungen (z.B. bei Zuckerkranken) geben.

In der Orthopädie können textile Drucksensoren der hier angesprochenen Art dazu dienen, die Druckverteilung eines Fußes auf einer Schuhsohle zu erfassen. Auf der Grundlage der erfassten Druckverteilung kann die Schuhsohle, insbesondere durch Anfertigung einer individuellen Einlegesohle dann so angepasst werden, dass Druckspitzen zwischen dem Fuß und dem Schuhmaterial vermieden werden. Insbesondere bei Sportschuhen oder Skischuhen ist eine derartige individuelle Anpassung von Schuhen von besonderem Interesse. Beispielsweise ist es für den Hersteller von Sportschuhen interessant, wie sich bei Geh- oder Laufbewegungen die wirkenden Kräfte über die Fußfläche des Schuhträgers verteilen. Entsprechend können die Schuhe je nach Anwendungsgebiet optimiert werden. Federnde oder stützende Elemente können entsprechend im Schuh angeordnet werden. Daraus ergibt sich eine optimale Anpassung des Schuhs an den Träger, wobei die individuelle Kraftverteilung, die abhängig von der Anatomie des Schuhträgers und dessen Bewegungsabläufen auftritt, berücksichtigt wird. Im Bereich des Sports kann ein textiler Drucksensor weiterhin bei der Herstellung von Skiern zur Erfassung der Druckverteilung dienen, um eine Kernkonstruktion entsprechend anpassen zu können. Die Anwendung eines textilen Drucksensors ist grundsätzlich auch zur Optimierung von Fahrradsatteln oder Reitersatteln denkbar.

Weitere Anwendungsgebiete sind druckempfindliche Bodenbeläge, um eine Bewegung eines Objekts oder eine Belastung bzw. eine Belastungsänderung eines Bodens in einem Gebäude differenziert feststellen zu können. Weitere Anwendung finden textile Drucksensoren im Bereich der künstlichen Häute für Roboter zur Nachbildung taktiler Sinne. Auch für Sitzflächen jeglicher Art, insbesondere adaptive Sitze für Flugzeuge, Kraftfahrzeuge, Führerstände oder im privaten Bereich, wie beispielsweise für Bürostühle, werden textile Drucksensoren verwendet. Auch im Bereich der medizinischen Diagnostik finden textile Drucksensoren zur Messung der flächigen Verteilung von Druckkräften Anwendung. Ein weiteres Anwendungsgebiet ist die Messung von Muskelkraft, insbesondere von Ringmuskeln an oder in Körperöffnungen. Weiterhin ist eine Anwendung von textilen Drucksensoren im Bereich der Videospiel-Konsolen denkbar, insbesondere zur Erfassung einer Position und Bewegungen einer Person und Umsetzung in entsprechende Bewegungen von Spielfiguren oder -elementen auf einem Bildschirm. Auch zur Personen-Identifikation können textile Drucksensoren eingesetzt werden, wobei die individuellen Druckverteilungsmuster beispielsweise einer Hand oder eines Fußes zur eindeutigen Identifikation herangezogen werden.

Zusammenfassend können textile Drucksensoren der hier angesprochenen Art überall dort zum Einsatz kommen, wo allgemein Objekte und insbesondere Körperteile einer Person auf einem im Wesentlichen festen Untergrund aufliegen. Viele Objekte und insbesondere Körperteile, deren Druckverteilung auf einem Untergrund gemessen werden soll, sind jedoch nicht eben sondern unregelmäßig mit Erhebungen und Vertiefungen geformt. Eine gute Anpassbarkeit an unebene Objekte sowie eine erhebliche Robustheit bei einer Belastung durch solche Objekte stellen damit ein entscheidendes Qualitätskriterium von textilen Drucksensoren dar. Es hat sich jedoch gezeigt, dass die bekannten textilen Drucksensoren zur kapazitiven Messung einer Druckverteilung dieses Qualitätskriterium nur in begrenztem Maße erfüllen.

Aufgabe der vorliegenden Erfindung ist es daher, einen textilen Drucksensor zu schaffen, der eine gute Anpassbarkeit an unebene Flächen und Objekte sowie eine erhebliche Robustheit bei einer Belastung durch Objekte aufweist.

Zur Lösung der oben genannten Aufgabe wird ein textiler Drucksensor mit den Merkmalen des Anspruchs 1 vorgeschlagen. Der textile Drucksensor dient zur kapazitiven Messung einer Druckverteilung eines beliebig geformten Objekts, insbesondere eines Körperteils, auf einer Fläche und weist eine erste zumindest bereichsweise leitfähige Struktur und eine zweite zumindest bereichsweise leitfähige Struktur auf, wobei die erste und die zweite zumindest bereichsweise leitfähige Struktur durch ein dielektrisches Zwischenelement voneinander getrennt sind, und wobei leitfähige Bereiche der ersten Struktur mit gegenüberliegenden leitfähigen Bereichen der zweiten Struktur Kondensatoren ausbilden. Der textile Drucksensor zeichnet sich dadurch aus, dass die erste und/oder die zweite zumindest bereichsweise leitfähige Struktur als Gestricke ausgebildet ist.

An dieser Stelle sei erwähnt, dass im Nachfolgenden unter "Gestricke" auch "Gewirke", gestickte oder gehäkelte Ware zu verstehen ist, also (Maschen-) Ware, bei der einer oder mehrere Fäden in und miteinander verschlungen sind. Ein loses Vlies fällt also auch darunter. Weiterhin sei erwähnt, dass unter "Faden" nicht nur Gebilde aus verbundenen/verdrehten Fasern sondern auch Monofilamente zu verstehen sind. Ebenso ist zu erwähnen, dass "Fäden" dann, wenn sie leitend sein sollen, aus leitenden und nichtleitenden Fasern aufgebaut sein können.

Ein wesentlicher Punkt der Erfindung liegt somit darin, dass der textile Drucksensor gemäß der Erfindung auch die Druckverteilung von uneben geformten Objekten optimal messen kann, ohne dass die Gefahr eines Risses oder dergleichen Beschädigung in einer Zugrichtung des textilen Drucksensors besteht. Der Drucksensor ist durch die Verwendung eines Gestrickes besonders robust ausgebildet ist, da das Gestricke, im Gegensatz zu einem Gewebe, in alle Richtungen dehnbar ist. Ein Gestricke zeichnet sich in der Regel dadurch aus, dass es bereichsweise aus einem einzigen Faden oder Garn hergestellt ist, wobei der Faden oder das Garn in maschenförmigen Schleifen (Maschen) verschlungen ist. Ein Gestricke weist also, im Gegensatz zu einem Kett- und Schussfäden aufweisenden Gewebe, Maschenreihen (und Maschenspalten) auf, die ein zusammenhängendes Gestricke (oder Gewirke) ergeben. Das Gestricke kann sich dadurch besonders gut an einen Körper bzw. dessen Form anpassen. Ein weiterer Vorteil des erfindungsgemäßen Drucksensors sind die vielfältigen Formen, die der Sensor durch die Verwendung eines Gestrickes annehmen kann. Insbesondere lässt sich der textile Drucksensor auch in einer dreidimensionalen Form (z.B. als Strumpf oder Handschuh) stricken, was wiederum mit anderen Materialien, selbst wenn sie eine hohe Dehnbarkeit in allen Richtungen aufweisen, nicht ohne Weiteres möglich ist. Dieser Vorteil wird durch die Ausbildung der ersten und/oder der zweiten zumindest bereichsweise leitfähigen Struktur als Gestricke erreicht. Das Gestricke ist besonders vorteilhaft, weil es im Gegensatz zu einem Gewebe auch aus nur einem einzigen Faden oder Garn ausgebildet sein kann, wobei der Faden oder Garn sehr dünn sein und eine hohe Maschendichte aufweisen kann. Textile Drucksensoren, die gemäß der Erfindung ein Gestricke aufweisen, können somit für jeden erdenklichen individuellen Einsatz, insbesondere auch zur Umhüllung von komplizierten dreidimensionalen Formen zum Einsatz kommen. Beispielsweise ist es denkbar, ein schlauchförmiges oder gar kugelförmiges Gestricke für einen textilen Drucksensor zur kapazitiven Messung einer Druckverteilung zu stricken. Durch das Maschen aufweisende Gestricke ist der textile Drucksensor sowohl in der Längs- als auch in der Querrichtung allein schon aufgrund der gekrümmten Fadenverläufe elastisch ausgebildet und weist somit gegenüber anderen Materialien wie beispielsweise einem Gewebe eine deutlich erhöhte Dehnbarkeit bzw. Flexibilität auf, wobei auch die Reißfestigkeit des erfindungsgemäßen Sensors deutlich erhöht ist. Ein Gestricke ist darüber hinaus besonders weich und anschmiegsam und kann somit in vorteilhafter Weise für Bekleidungsstücke wie Socken, Hosen oder Pullover oder auch als Bezug eines Autositzes zum Einsatz kommen. Darüber hinaus neigt das Gestricke nicht zu Knittern und ist somit für den Einsatz in textilen Drucksensoren besonders gut geeignet.

Erfindungsgemäß ist vorgesehen, dass die erste und/oder die zweite zumindest bereichsweise leitfähige Struktur als Gestricke ausgebildet sind. Beispielsweise ist es denkbar, nur die dem zu messenden Objekt zugewandte Oberseite des textilen Drucksensors, d.h. die erste Struktur, aus einem Gestricke herzustellen, um den Komfort des textilen Drucksensors insbesondere im Bereich von Bekleidungsstücken zu erhöhen. Es kann jedoch auch vorgesehen sein, dass lediglich die vom Objekt abgewandte Unterseite des textilen Drucksensors, d.h. die zweite Struktur als Gestricke ausgebildet ist, beispielsweise um die Anpassbarkeit des Sensors an einen Untergrund zu erhöhen. Besonders bevorzugt wird eine Ausführungsform, bei der sowohl die erste als auch die zweite Struktur als Gestricke ausgebildet ist. Dadurch wird eine maximale Flexibilität des Sensors erzielt.

Weiterhin ist es möglich, die leitfähigen Strukturen auch durch additive Verfahren, also das Aufbringen von leitfähigem Material (z.B. Galvanisieren, bedrucken, bedampfen) von nicht leitendem Material zu erzeugen. Umgekehrt können nichtleitende Bereiche durch subtraktive Verfahren erzeugt werden, z.B. durch ätzen oder schneiden.

Zur Erzeugung eines leitfähigen Bereichs bzw. mehrerer leitfähiger Bereiche in der ersten und/oder in der zweiten Struktur, sind in das Gestricke vorzugsweise leitfähige Fäden während des Strickens eingearbeitet, d.h. eingestrickt. Die leitfähigen Bereiche können insbesondere in Form von abgetrennten, d.h. voneinander isolierten Bahnen eines leitfähigen Fadens oder Garns ausgebildet sein. Mehrere voneinander isolierte, leitfähige Bereiche des Gestrickes können jeweils mehrere benachbarte Maschenreihen eines leitfähigen Fadens, Filaments oder Garns umfassen. Moderne industrielle Strickmaschinen ermöglichen die Herstellung nahezu beliebiger Muster und Kombinationen von Mustern, die das Gestricke, insbesondere zur Ausbildung von leitfähigen Bereichen aufweisen kann. Moderne Strickmaschinen ermöglichen es darüber hinaus, elektrisch leitfähige Fäden oder Garne beispielsweise aus Kohlefaser, Edelstahl, Gold, Silber oder Messing, insbesondere in Form von sogenannten "technischen Gestricken", zu verarbeiten. Hierbei können die Fäden oder Garne selbst elastisch sein, müssen dies aber nicht, da die Maschen selbst nachgiebige Strukturen bilden.

Die leitfähigen Bahnen der ersten Struktur sind unter einem Winkel, beispielsweise von 90 Grad, relativ zu den leitfähigen Bahnen der zweiten Struktur angeordnet. Sofern die die Unterseite bildende zweite Struktur durchgehend leitfähig ausgebildet ist, kommt es auf die Anordnung der leitfähigen Bereiche der ersten Struktur relativ zu dem leitfähigen Bereich der zweiten Struktur nicht an. Allerdings ist es dann notwendig, eine inselartige bzw. matrixartige Anordnung von abgetrennten leitfähigen Bereichen der ersten Struktur vorzusehen, um die Ausbildung einer ausreichenden Anzahl von Kondensatoren zu gewährleisten.

Entscheidend ist, dass die erste und die zweite Struktur und insbesondere deren leitfähige Bereiche, so einander gegenüberliegend angeordnet sind, dass die leitfähigen Bereiche zur Ausbildung von Kondensatoren Überschneidungsflächen bilden. Hierzu kann natürlich auch vorgesehen sein, dass die leitfähigen Bahnen einen spitzeren Winkel als 90° zueinander aufweisen, sofern sowohl die erste als auch die zweite Struktur leitfähige, gegeneinander isolierte Bahnen aufweisen. Die leitfähigen Bereiche weisen entsprechende Verbindungen zur Auswertelektronik auf, die ebenfalls in die erste und/oder die zweite Struktur eingestrickt sein können.

Das Gestricke ermöglicht es weiterhin in vorteilhafter Weise, Anschlüsse in die erste und zweite Struktur einzuarbeiten bzw. einzustricken, die zur Verbindung leitfähiger Bereiche mit einer Energieversorgungsquelle und mit einer entsprechenden Auswertelektronik dienen. Die Anschlüsse können beispielsweise in Form von über den Stoffrand des textilen Drucksensors hinausragenden noppen- oder zapfenförmigen Anschlusselementen ausgebildet sein, die vorzugsweise Teil des Gestrickes sind.

Weiterhin können Zuleitungen/Anschlüsse durch kleben, löten, schweißen, klemmen oder aber nähen erzeugt werden.

Wie gesagt sind die erste und die zweite zumindest bereichsweise leitfähige Struktur vorzugsweise einander gegenüberliegend angeordnet. Weiterhin ist das dielektrische Zwischenelement aus einem reversibel komprimierbaren Material ausgebildet, so dass nach einem Zusammendrücken des textilen Drucksensors in einem Bereich der textile Drucksensor in diesem Bereich wieder in seinen Ausgangszustand zurückkehren kann. Das dielektrische Zwischenelement kann ebenfalls als Gestricke ausgebildet sein. Besonders geeignet ist hier ein Wirkverfahren auf einer Doppelraschelmaschine, wobei das dielektrische Zwischenelement durch entsprechend ausgebildete Polfäden erzeugt wird. In diesem Fall ist das Gestricke bzw. Gewirke vorzugsweise aus einem reversibel komprimierbaren Material ausgebildet, d.h. mit einem entsprechenden Faden oder Garn gestrickt. Es kann auch vorgesehen sein, dass das dielektrische Zwischenelement alternativ oder zusätzlich integrierte Rückstellelemente im Bereich der Überschneidungsflächen der leitfähigen Bereiche aufweist. Diese integrierten Rückstellelemente können in das Gestricke eingearbeitet bzw. eingestrickt sein. Auf diese Weise wird die auf dem Gestricke beruhende Flexibilität und Elastizität des textilen Drucksensors nicht durch das isolierende Material beeinträchtigt. Beispielsweise ist es denkbar, die integrierten Rückstellelemente ausschließlich in mehreren Maschenreihen auszubilden, die aus einem reversibel komprimierbaren und isolierenden Garn oder Filament bestehen und die im Bereich der Überschneidungsflächen der leitenden Bereiche angeordnet sind. Weiterhin ist es denkbar, Rückstellelemente lediglich im Bereich inselförmiger Ausprägungen, umfassend mehrere Maschen des Gestrickes, vorzusehen.

Bei einer Ausführungsform der Erfindung kann vorgesehen sein, dass lediglich eine der beiden zumindest bereichsweise leitfähigen Strukturen aus einem Gestricke ausgebildet ist. Beispielsweise ist es denkbar, eine der beiden Strukturen aus einem durchgängig leitfähigen Material auszubilden und nur die weitere, gegenüberliegende Struktur aus einem Gestricke mit integrierten, d.h. angestrickten leitfähigen Bereichen auszubilden. Weiterhin ist es denkbar, die leitfähigen Bereiche bei einer ersten oder der zweiten Struktur unmittelbar auf das dielektrische Zwischenelement aufzubringen, insbesondere aufzudrucken oder anzukleben, während die andere Struktur als Gestricke mit gestrickten leitenden Bereichen ausgebildet ist.

Bei einer weiteren Ausführungsform kann sowohl die erste als auch die zweite Struktur aus einem Gestricke bestehen, welches eingearbeitete leitfähige Bereiche aufweist, die Überschneidungsflächen zur Ausbildung von Kondensatoren bilden. Das dielektrische Zwischenelement kann dann beispielsweise als Schaumstoffschicht oder dergleichen reversibel komprimierbares Element ausgebildet sein.

Bei einer weiteren Ausführungsform der Erfindung kann vorgesehen sein, dass sowohl die erste und die zweite Struktur mit den leitfähigen Bereichen als auch das dielektrische Zwischenelement als Gestricke ausgebildet sind. Während in das Gestricke der ersten und zweiten zumindest bereichsweise leitenden Struktur leitfähige Bereiche eingestrickt sind, sind derartige leitende Bereiche bei der dielektrischen Zwischenschicht nicht vorgesehen.

Die Schichten des textilen Drucksensors können beispielsweise miteinander durch einen isolierenden Faden vernäht sein. Dies gilt auch dann, wenn ein oder mehrere Schichten des textilen Drucksensors nicht als Gestricke ausgebildet sind.

Denkbar ist im Übrigen eine Ausführungsform der Erfindung, bei der der Drucksensor aus einem einzigen zusammenhängenden bahnartigen Gestricke ausgebildet ist. Beispielsweise ist es denkbar, das bahnartige Gestricke so s- oder z-förmig zu falten und anschließend zu verbinden, insbesondere zu vernähen, dass ein dreilagiger mattenförmiger textiler Drucksensor entsteht. Das bahnartige Gestricke umfasst dann vorzugsweise leitfähige, über einen isolierenden Faden verbundene Bahnen, die so ausgebildet und angeordnet sind, dass nach dem Falten Überschneidungsbereiche zwischen der ersten und der zweiten Struktur zur Ausbildung von Kondensatoren gebildet werden, während die dielektrische Zwischenschicht keine leitenden Bereiche aufweist. Vielmehr können in die Zwischenschicht Rückstellelemente eingebracht werden oder der die Zwischenschicht bildende Teil des zusammenhängenden bahnartigen Gestrickes kann aus einem reversibel komprimierbaren Material gestrickt sein.

Es kann auch vorgesehen sein, lediglich die erste und die zweite Struktur des textilen Drucksensors einstückig aus einem einzigen zusammenhängenden bahnartigen Gestricke mit (gestrickten) leitfähigen und zwischen diesen (getrickten) isolierenden Maschenreihen Bereichen oder Bahnen auszubilden. Zwischen den beiden einander gegenüberliegenden gestrickten Strukturen kann dann ein separates dielektrisches Zwischenelement eingefügt werden, welches mit der ersten und der zweiten Struktur verbunden wird.

Insgesamt zeigt sich, dass der textile Drucksensor gemäß der Erfindung zumindest teilweise aus einem Gestricke besteht, was erhebliche Vorteile gegenüber Sensoren aus einem anderen Material, insbesondere aus einem Gewebe oder einer Folie, hat. In einem Gestricke oder Gewirke ist es beispielsweise in vorteilhafter Weise möglich, ohne Verlust der Elastizität des textilen Drucksensors, Bahnen aus einem leitfähigen Faden oder Garn von dem übrigen, nichtleitenden Gestricke abzutrennen. Das Gestricke umfasst dann quasi gestrickte Elektroden. Bei textilen Drucksensoren, die aufgeklebte Elektroden aufweisen, ergibt sich insbesondere in Bezug auf die Elastizität des Sensors eine erhebliche Verschlechterung.

Das Gestricke gemäß der vorliegenden Erfindung umfasst vorzugsweise sich abwechselnde Gruppen von Maschenreihen aus einem leitfähigen und einem nichtleitfähigen Faden oder Garn. Durch diese Anordnung wechseln sich leitende und nichtleitende Bahnen ab, wobei eine Bahn jeweils mehrere Maschenreihen und mehrere Maschenspalten umfassen kann.

Die Anzahl der Kondensatoren des textilen Drucksensors hängt davon ab, wie viele Überschneidungsbereiche von leitenden Bereichen bzw. Elektroden vorgesehen sind. Es versteht sich, dass die Anzahl der Kondensatoren größer ist, je größer die Anzahl der leitenden Bereiche und damit Überschneidungsbereiche ist. Die Anzahl und Verteilung der Kondensatoren und damit die Genauigkeit der Druckverteilungsmessung hängt folglich von dem "Strickmuster" der Elektroden, d.h. der leitenden Bereiche ab.

Aus Obigem geht hervor, dass die Erfindung auch ein Verfahren zur Herstellung eines textilen Drucksensors betrifft. Vom Aspekt der Herstellung ist nämlich das Stricken oder Wirken eine besonders vorteilhafte Herstellungsweise, weil sehr komplizierte Strukturen durch Strickmaschinen/Wirkmaschinen "automatisiert" hergestellt werden können. Es können nämlich leitfähige Bereiche zur Bildung von Kondensator-Elektroden und/oder Anschlussbahnen und/oder isolierende Bereiche zum isolierten Verbinden der Kondensator-Elektroden miteinander gestrickt werden, ohne dass besonders viele diffizile handarbeitliche Schritte durchgeführt werden müssen. Insbesondere dann, wenn die isolierenden Bereiche an die leitfähigen Bereiche angestrickt werden, entstehen optimale mechanische Verbindungen ohne die Notwendigkeit, Schnitte oder Klebarbeiten durchführen zu müssen, wie dies bisher bei Geweben und noch mehr bei Folienstrukturen der Fall war.

Weiterhin können auch mindestens zwischen einander gegenüberliegenden, Kondensatoren bildenden leitfähigen Bereiche weitere isolierende Bereiche zur Bildung komprimierbarer Kondensator-Dielektrika eingestrickt werden, welche die leitfähigen Bereiche miteinander verbinden und in diese eingestrickt sind. Es wird also sozusagen dreidimensional gestrickt.

Die hier als erfindungswesentlich beanspruchte Verwendung von Gestricken oder Gewirken hat auch den weiteren Vorteil, dass allein durch die Maschenstruktur eines Gestrickes oder Gewirkes relativ weiche und vor Allem auch dehnbare Gebilde hergestellt werden können, ohne dass hierzu dehnbare Garne oder Fäden verwendet werden müssten. Es können also sogar (wie oben angedeutet) Monofilamente aus Metall bzw. aus Kunststoff (für die leitenden bzw. die nicht leitenden Bereiche) verwendet werden, wenn die Strickart so gewählt wird, dass die benötigte Elastizität des gesamten Gestrickes gewährleistet ist.

Wie aus Obigem hervorgeht, liegt ein Vorteil eines textilen, insbesondere eines aus Maschenware hergestellten kapazitiven Drucksensors in seiner Dehnbarkeit. Dadurch kann die Fläche eines Kondensatorelements um bis zu 100% zunehmen. Allerdings hat dies auch nachteilige Effekte:
a) Wird aus dem Druck der Elemente die resultierende Kraft nach der Formel F=a*p (a=Fläche; p=Druck), so ist die Kraft F fehlerbehaftet, da p gemessen werden werden, a aber nun keine Konstante mehr ist.
b) Da eine Zunahme der Fläche die Kapazität des Messkondensators bewirkt, ist der gemessene Druck höher ist der tatsächlich aufgebrachte.
c) Bei Dehnung des druckaufnehmenden Dielektrikums kann, abhängig von der Ausführung des Dielektrikums, dessen Dicke abnehmen, was wie zuvor beschrieben, den gemessenen Druckwert erhöht.

Die Effekte b) und c) können a teilweise kompensieren, also die Genauigkeit der Kraftmessung verbessern. Der Fehler der Druckmessung kann so nicht kompensiert werden.

Nun kann man die Effekte der Dehnung beheben:
A: Getrennte Messung der Dehnung
   a. Über der druck- und dehnungsempfindlichen Schicht wird eine zweite Schicht angeordnet, die nur auf Dehnung anspricht. Das wird erreicht durch ein inkompressibles und elastisches Dielektrikum.
   b. Weiterhin kann man druckempfindliche und ausschließlich dehnungsempfindliche Sensorelemente in einer einzigen Sensormatrix anordnen, indem das Dielektrikum wechselweise druckempfindlich oder ausschließlich dehnungsempfindlich ausgebildet ist. In diesem Fall muss die örtliche Auflösung der Matrix so hoch sein, dass die Druck- oder Dehnungsunterschiede zwischen benachbarten Sensorelementen ein bestimmtes Maß nicht überschreiten.
   c. Durch einen Kalibrierungs-Algorithmus kann der wahre Druck bestimmt werden.
   d. Über die Messung der Dehnung kann, insbesondere wenn die Dehnung in x- und y-Richtung getrennt bestimmt wird, die dreidimensionale Verformung der Sensormatrix berechnet werden.
B: Ausbildung als linienförmige Kondensatorelektroden
   a. Die Kondensatorplatten werden nicht als homogene Flächen betrachtet sondern als getrennt parallel laufende leitfähige Fäden, die gegeneinander isoliert und deren Abstand groß ist im Vergleich zu den Gegenelektroden, so dass die elektrischen Felder benachbarter Fäden sich nur wenig überlagern. Verändert sich der Abstand der Fäden bei Dehnung, ändern sich die Summen der durch die Einzelfäden gebildeten Kapazitäten nur geringfügig.
   b. Eine solche Anordnung kann leicht durch textile Techniken (Gewebe) gefertigt werden.
   c. Ist die Gegenelektrode flächenförmig ausgebildet, bleibt die Kapazität bei Dehnung quer zur Fadenrichtung konstant und nimmt bei Dehnung in Fadenrichtung zu.
   d. Ist die Gegenelektrode ebenfalls aus isolierten Fäden aufgebaut, so gibt es zwei Fälle, wobei die Kondensatoren jeweils linienförmig ausgebildet sind.
   e. Die Fäden verlaufen parallel zur ersten Elektrode.
      1. Die fadenförmige Elektroden liegen so, dass sich die Fäden genau gegenüberstehen. Der Abstand zwischen den Elektroden ist minimal. Bei Dehnung quer zum Faden: Die Kapazität ist konstant. Längs des Fadens: Die Kapazität steigt (wie oben c.).
      2. Wie unter Ziffer 1, aber die Fäden sind so versetzt, dass sich ein maximaler Abstand zwischen den Elektroden ergibt.
         Dehnung quer zum Faden: Die Kapazität nimmt ab.
         Dehnung längs zum Faden: Die Kapazität nimmt zu.
      3. Die Elektroden sind um 90 Grad gegeneinander verdreht angeordnet. Die Kondensatoren sind eher punktförmig ausgebildet.
      Bei Dehnung in jeder Richtung bleibt die Kapazität konstant.

Die Erfindung wird im Folgenden anhand der Zeichnung näher erläutert. Es zeigen:
- Fig. 1: eine schematische Draufsicht auf eine als Gestricke ausgebildete zumindest bereichsweise leitfähige Struktur gemäß der Erfindung;
- Fig. 2: eine schematische Draufsicht auf eine als Gestricke ausgebildete, zumindest bereichsweise leitfähige Struktur mit Anschlüssen;
- Fig. 3: eine schematische Schnittdarstellung eines textilen Drucksensors gemäß der Erfindung;
- Fig. 4: eine schematische Draufsicht auf abwechselnde Gruppen von Maschenreihen aus leitfähigen und nichtleitfähigen Fäden gemäß der Erfindung;
- Fig. 5: eine schematische Draufsicht auf einen textilen Drucksensor gemäß der Erfindung;
- Fig.6: eine weitere Ausführungsform in einer Darstellung nach Fig. 5 und
- Fig. 7: einen aus dem Stand der Technik bekannten textilen Drucksensor.

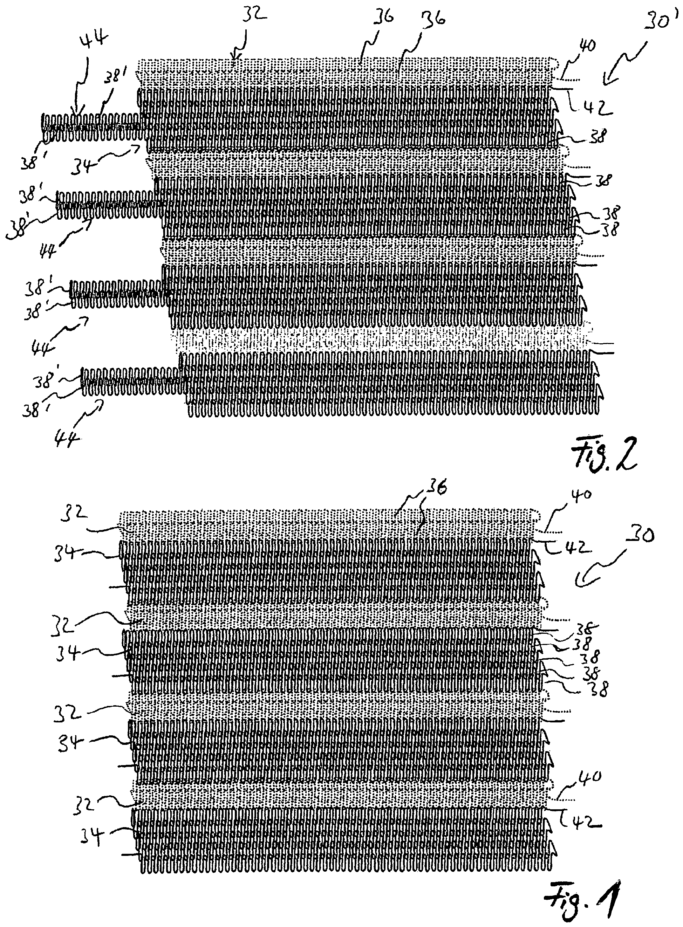

Fig. 1 zeigt eine schematische Draufsicht auf eine als Gestricke ausgebildete zumindest bereichsweise elektrisch leitfähige Struktur 30. Die in Fig. 1 gezeigte zumindest bereichsweise leitfähige Struktur 30 kann sowohl die erste als auch die zweite in Fig. 7 gezeigte zumindest bereichsweise leitfähige Struktur 3 oder 5 bilden. Die zumindest bereichsweise leitfähige Struktur 30 umfasst in der Fig. 1 punktiert dargestellte elektrisch nichtleitfähige Bereiche 32 sowie dunkel dargestellte elektrisch leitfähige Bereiche 34. In der Ausführungsform nach Fig. 1 umfasst jeder nichtleitfähige Bereich 32 zwei Maschenreihen 36 eines nichtleitenden Filamentes oder Fadens. Die leitfähigen Bereiche 34 umfassen hingegen jeweils fünf Maschenreihen 38. Es versteht sich, dass die Zahl der Maschenreihen 36, 38 der nichtleitfähigen bzw. leitfähigen Bereiche 32, 34 unterschiedlich sein kann. Vorzugsweise sind die nichtleitfähigen Bahnen besonders schmal ausgebildet und können also lediglich eine einzige Maschenreihe 36 aufweisen.

Die Fig. 1 macht deutlich, dass jeder Bereich 32 und 34 mit einem einzigen Faden 40 bzw. 42 gestrickt ist. Dabei wird ein leitfähiger Bereich 34 unmittelbar an einen nichtleitfähigen Bereich 32 angestrickt. Entsprechend wird auch ein nichtleitfähiger Bereich 32 mit einem Faden 40 an einen Faden 42 eines leitfähigen Bereichs 34 angestrickt. Bei dem Faden 40 handelt es sich dabei um einen Faden aus einem elektrisch nichtleitenden Material, während es sich bei dem Faden 42 um einen Faden aus einem elektrisch leitenden Material handelt. Entsprechend bilden die elektrisch leitfähigen Bereiche 34 die Elektroden der Struktur 30. Der Faden 42 kann auch beides, elektrisch leitende und nichtleitende Fasern umfassen.

Die Fig. 2 zeigt eine schematische Draufsicht auf eine zumindest bereichsweise leitfähige Struktur 30', die zusätzlich mit Anschlüssen 44 versehen ist. Die Anschlüsse 44 dienen zur Verbindung der leitfähigen Bereiche 34 mit einer Energiezufuhr und/oder einer Auswertelektronik. Die Anschlüsse 44 sind dabei mittels des Fadens 42 gestrickt, der die leitfähigen Bereiche 34 bildet. In der Fig. 2 sind die Anschlüsse 44 durch zwei Maschenreihen 38' gebildet, die länger sind als die übrigen Maschenreihen 38 der leitfähigen Bereiche 34, und die auch länger sind als die Maschenreihen 36 der nichtleitfähigen Bereiche 32. Die Maschenreihen 36 der nichtleitfähigen Bereiche 32 und die Maschenreihen 38 der leitfähigen Bereiche 34 sind im Übrigen gleich lang ausgebildet. Die Anschlüsse 44 ragen somit über den Rand der gestrickten Struktur 30' hinaus. Alternativ kann vorgesehen sein, die Anschlüsse 44 separat auszubilden, insbesondere zu stricken und anschließend mit den leitfähigen Bereichen 34, insbesondere in einem Randbereich, zu verbinden, beispielsweise zu vernähen oder aber anzustricken.

Insgesamt zeigt sich, dass durch die abwechselnde gruppenartige Aneinanderreihung von Maschenreihen aus einem elektrisch leitfähigen und einem nichtleitfähigen Faden oder Garn abwechselnde leitfähige und nichtleitfähige Bahnen gebildet werden. Die leitfähigen Bahnen 34 sind folglich durch die nichtleitfähigen Bahnen elektrisch voneinander isoliert.

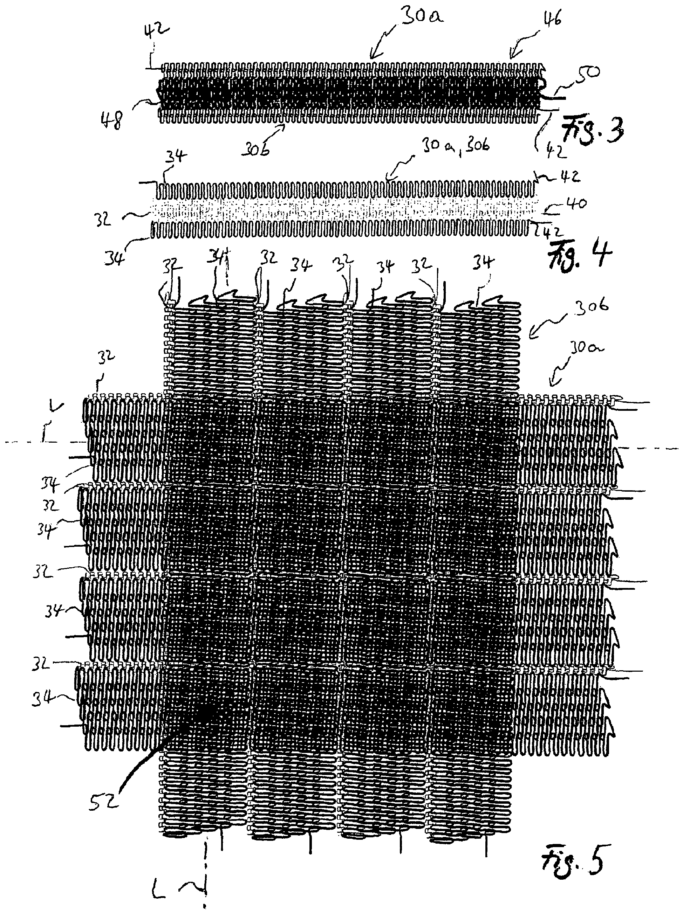

Die Fig. 3 zeigt eine schematische Schnittdarstellung eines textilen Drucksensors 46 gemäß der Erfindung. In der Ausführungsform nach Fig. 3 sind sowohl die erste, obere zumindest bereichsweise leitfähige Struktur 30a als auch die zweite, untere zumindest bereichsweise leitfähige Struktur 30b als Gestricke ausgebildet. Das Gestricke kann dabei, wie in den Fig. 1 und 2 gezeigt, ausgebildet sein. Zwischen der ersten und der zweiten zumindest bereichsweise leitfähigen Struktur 30a und 30b ist ein Zwischenelement 48 angeordnet, welches ebenfalls als Gestricke ausgebildet und elastisch komprimierbar ist. Auch das Zwischenelement 48 ist aus einem einzelnen Faden 50 ausgebildet. Der Faden 50 ist zu einem dreidimensionalen Gestricke gestrickt, welches also nicht nur Maschenreihen in einer einzigen Ebene aufweist, sondern das vielmehr Maschenreihen in mehreren miteinander verstrickten Ebenen aufweist. Auf diese Weise kann ein mehrere Maschenebenen aufweisendes, gestricktes dielektrisches Zwischenelement 48 geschaffen werden, welches reversibel komprimierbar ausgebildet ist. Dazu muss der Faden 50 noch nicht einmal dehnbar, sondern nur flexibel sein, da die Maschenstruktur die Dehn- und Komprimierbarkeit der Anordnung gewährleistet. Dies gilt übrigens auch für die Fäden 40 und 42 der leitenden bzw. isolierenden Bereiche. Zur Herstellung eines solchen Gebildes eignen sich besonders Wirkmaschinen (Doppelraschelmaschinen), auf denen Abstandsgewirke hergestellt werden und der Polfaden das isolierende und elastische Zwischenelement 48 bildet.

Vorzugsweise ist der textile Drucksensor 46 so aufgebaut, dass das Zwischenelement 48 mittels des Fadens 50 an die erste zumindest bereichsweise leitfähige Struktur 30a unmittelbar angestrickt ist. Der Faden 50 wird hierzu durch die Maschen der Struktur 30a geführt. Entsprechend ist die zweite zumindest bereichsweise leitfähige Struktur 30b an das Zwischenelement 48 mittels des Fadens 42 gestrickt. Auf diese Weise entsteht ein dreidimensionaler textiler Drucksensor 46, der vollständig aus einem zusammenhängenden Gestricke (oder Gewirke) ausgebildet ist. Alternativ kann vorgesehen sein, dass die erste Struktur 30a, die zweite Struktur 30b und das Zwischenelement 48 als separate Gestricke ausgebildet sind, die miteinander vernäht oder verklebt sind, statt als zusammenhängendes Gestricke ausgebildet zu sein.

In der Fig. 4 ist eine schematische Draufsicht auf einen Ausschnitt einer zumindest bereichsweise leitfähigen Struktur 30a bzw. 30b gezeigt. Die Fig. 4 macht deutlich, wie die leitfähigen Bereiche 34 mittels des Fadens 42 an den nichtleitfähigen Bereich 32 angestrickt sind. Hierzu sind die Fäden 42 mit dem Faden 40 so verstrickt, dass miteinander verbundene Maschenreihen entstehen.

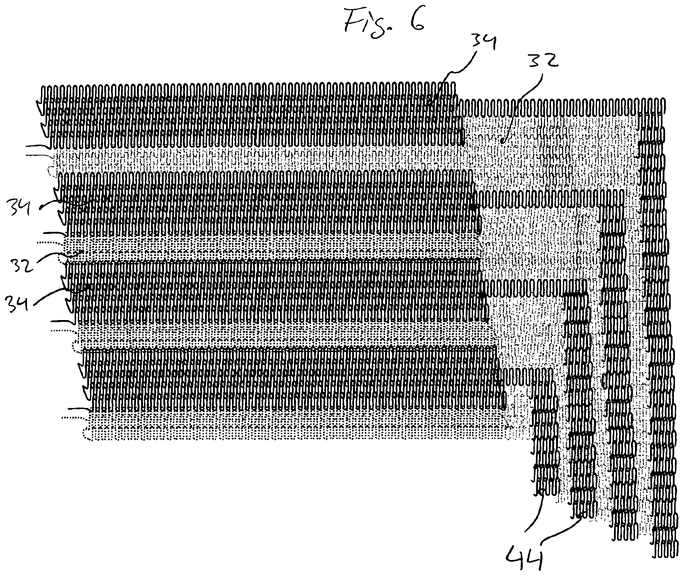

Fig. 5 zeigt eine Anordnung von zwei zumindest bereichsweise leitfähig ausgebildeten Strukturen 30a und 30b zur Realisierung eines textilen Drucksensors 46. In der Fig. 6 sind die leitfähigen Bereiche 32 der Strukturen 30a und 30b so zueinander angeordnet, dass ihre Längsachsen L im Wesentlichen rechtwinklig zueinander angeordnet sind. Es versteht sich, dass zwischen den Strukturen 30a und 30b ein in Fig. 3 gezeigtes und in Fig. 5 nicht erkennbares dielektrisches Zwischenelement 48 angeordnet sein muss, um den kapazitiven textilen Drucksensor 46 zu realisieren.

Die Fig. 5 macht deutlich, dass durch die im Wesentlichen rechtwinklige Anordnung der bahnartigen nichtleitfähigen Bereiche 34 der Strukturen 30a und 30b matrixförmig angeordnete, rechteckige Überschneidungsbereiche 52 resultieren, die jeweils einen Kondensator ausbilden. Auf diese Weise kann abhängig von der Anzahl der leitfähigen Bereiche 34 die Anzahl der ausgebildeten Kondensatoren variieren.

In Fig. 6 ist eine weitere Ausführungsform der Erfindung gezeigt, die ähnlich der nach Fig. 2 ist. Hierbei sind die leitfähigen Bereiche 34 miteinander über angestrickte nichtleitfähige Bereiche 32 zur Bildung der Kondensatorbahnen verbunden. Die Anschlüsse 44 sind ebenfalls über nichtleitfähige Bereiche 32 durch Anstricken verbunden, so dass ein insgesamt sehr stabiles Gebilde entsteht. Es sei hierbei aber auch darauf hingewiesen, dass die in den Zeichnungen angegebenen Dimensionen und Dimensionsverhältnisse lediglich aus Gründen der zeichnerischen Darstellung so gewählt wurden. Im Allgemeinen sind natürlich die Anschlüsse 44 durch sehr viel schmalere Bahnen gebildet als die eigentlichen Kondensatorflächen, welche durch die leitenden Bereiche 34 gebildet sind.

Insgesamt schafft die vorliegende Erfindung einen vorteilhaften Drucksensor, der mittels eines Gestrickes flexibel ausgebildet ist und vielseitig einsetzbar ist. Darüber hinaus ist der Drucksensor gemäß der Erfindung besonders robust und komfortabel, insbesondere bei einem Einsatz des Drucksensors in Kleidungsstücken.

### Bezuaszeichenliste

- 1: Textiler Drucksensor
- 3: Erste bereichsweise leitfähige Struktur
- 5: Zweite bereichsweise leitfähige Struktur
- 7: Dielektrisches Zwischenelement
- 9: Leitfähige Bereiche
- 11: Überschneidungspunkte
- 13: Druckkraft
- 30, 30': Zumindest bereichsweise leitfähige Struktur
- 30a: Erste zumindest bereichsweise leitfähige Struktur
- 30b: Zweite zumindest bereichsweise leitfähige Struktur
- 32: Nichtleitfähiger Bereich
- 34: Leitfähiger Bereich
- 36: Maschenreihen
- 38: Maschenreihen
- 40: Faden
- 42: Faden
- 44: Anschlüsse
- 46: Textiler Drucksensor
- 48: Zwischenelement
- 50: Faden
- 52: Überschneidungsbereiche
- C: Kondensator
- L: Längsrichtung

## Patentansprüche

1. Textiler Drucksensor zur kapazitiven Messung einer Druckverteilung von beliebig geformten Objekten, insbesondere Körperteilen, auf einer Fläche, aufweisend eine erste zumindest bereichsweise leitfähige Struktur (30a) und eine zweite zumindest bereichsweise leitfähige Struktur (30b), wobei die erste und die zweite zumindest bereichsweise leitfähige Struktur durch ein komprimierbares dielektrisches Zwischenelement (48) voneinander getrennt sind, und wobei leitfähige Bereiche (34) der ersten Struktur (30a) mit gegenüberliegenden leitfähigen Bereichen (34) der zweiten Struktur (30b) Kondensatoren ausbilden,
**dadurch gekennzeichnet, dass**
die erste und die zweite zumindest bereichsweise leitfähige Struktur (30a, 30b) als Gestricke ausgebildet sind und jeweils mehrere leitfähige Bereiche (34) umfassen, die durch angestrickte, nichtleitfähige Bereiche (32) miteinander verbunden sind.

2. Textiler Drucksensor nach Anspruch 1,
**dadurch gekennzeichnet, dass**
die leitfähigen Bereiche (34) des Gestrickes mehrere benachbarte Maschenreihen eines leitfähigen Garns oder leitfähiger Filamente umfassen.

3. Textiler Drucksensor nach Anspruch 1 oder 2,
**dadurch gekennzeichnet, dass**
die leitfähigen Bahnen der ersten Struktur (30a) unter einem Winkel zu leitfähigen Bahnen der zweiten Struktur (30b) angeordnet sind und die leitfähigen Bahnen Überschneidungsbereiche (52) ausbilden, die matrixförmig angeordnet sind.

4. Textiler Drucksensor nach einem der vorhergehenden Ansprüche,
**dadurch gekennzeichnet, dass**
das Gestricke eingearbeitete, insbesondere angestrickte, Anschlüsse (44) zur Verbindung leitfähiger Bereiche (34) mit einer elektrischen Auswerteinrichtung aufweist.

5. Textiler Drucksensor nach einem der vorhergehenden Ansprüche,
**dadurch gekennzeichnet, dass**
das dielektrische Zwischenelement (48) als vorzugsweise elastisch komprimierbares Gestricke ausgebildet ist.

6. Textiler Drucksensor nach einem der vorhergehenden Ansprüche,
**dadurch gekennzeichnet, dass**
das dielektrische Zwischenelement (48) integrierte Rückstellelemente im Bereich der Überschneidungspunkte aufweist.

7. Textiler Drucksensor nach einem der vorhergehenden Ansprüche, insbesondere nach Anspruch 6,
**dadurch gekennzeichnet, dass**
die integrierten Rückstellelemente in das Gestricke eingearbeitet sind.

8. Textiler Drucksensor nach einem der vorhergehenden Ansprüche, insbesondere nach Anspruch 7,
**dadurch gekennzeichnet, dass**
die integrierten Rückstellelemente mehrere Maschenreihen eines reversibel komprimierbaren Gestrickes umfassen und mindestens im Bereich der Überschneidungspunkte (52) angeordnet sind.

9. Textiler Drucksensor nach einem der vorhergehenden Ansprüche,
**dadurch gekennzeichnet, dass**
die erste und die zweite zumindest bereichsweise leitfähige Struktur (30a, 30b) und ggf. auch das dielektrische Zwischenelement (48) aus einem einzigen zusammenhängenden Gestricke ggf. aus mehreren, verschiedenen Garnen bestehen.

10. Verfahren zum Herstellen eines textilen Drucksensors zur kapazitiven Messung einer Druckverteilung zwischen einem Objekt und einer Fläche gemäß einem der Ansprüche 1 bis 9,
**dadurch gekennzeichnet, dass**
leitfähige Bereiche zur Bildung von Kondensator-Elektroden und/oder Anschlussbahnen und isolierende Bereiche zum isolierten Verbinden der Kondensator-Elektroden miteinander gestrickt werden.

11. Verfahren nach Anspruch 10,
**dadurch gekennzeichnet, dass**
die isolierenden Bereiche an die leitfähigen Bereiche angestrickt werden.

12. Verfahren nach einem Ansprüche 10 oder 11,
**dadurch gekennzeichnet, dass**
mindestens zwischen einander gegenüberliegenden, Kondensatoren bildenden leitfähigen Bereichen weitere, isolierende Bereiche zur Bildung komprimierbarer Kondensator-Dielektrika gestrickt, vorzugsweise die leitfähigen Bereiche miteinander verbindend, in diese eingestrickt werden.

## Claims

1. Textile pressure sensor for capacitively measuring a pressure distribution of objects of any desired shape, in particular body parts, on a surface, having a first structure (30a) which is conductive at least in regions and having a second structure (30b) which is conductive at least in regions, wherein the first and the second structures which are conductive at least in regions are separated from one another by a compressible dielectric intermediate element (48), and wherein conductive regions (34) of the first structure (30a) form capacitors with opposite conductive regions (34) of the second structure (30b),
**characterized in that**
the first and the second structures (30a, 30b) which are conductive at least in regions are in the form of knits and each comprise a plurality of conductive regions (34) which are connected to one another by knitted-on, non-conductive regions (32).

2. Textile pressure sensor according to Claim 1,
**characterized in that**
the conductive regions (34) of the knit comprise a plurality of adjacent stitch courses of a conductive yarn or conductive filaments.

3. Textile pressure sensor according to Claim 1 or 2,
**characterized in that**
the conductive webs of the first structure (30a) are arranged at an angle to conductive webs of the second structure (30b), and the conductive webs form overlap regions (52) which are arranged in the form of a matrix.

4. Textile pressure sensor according to one of the preceding claims,
**characterized in that**
the knit has incorporated, in particular knitted-on, connections (44) for connecting conductive regions (34) to an electrical evaluation device.

5. Textile pressure sensor according to one of the preceding claims,
**characterized in that**
the dielectric intermediate element (48) is in the form of a preferably elastically compressible knit.

6. Textile pressure sensor according to one of the preceding claims,
**characterized in that**
the dielectric intermediate element (48) has integrated return elements in the region of the overlap points.

7. Textile pressure sensor according to one of the preceding claims, in particular according to Claim 6,
**characterized in that**
the integrated return elements are incorporated into the knit.

8. Textile pressure sensor according to one of the preceding claims, in particular according to Claim 7,
**characterized in that**
the integrated return elements comprise a plurality of stitch courses of a reversibly compressible knit and are arranged at least in the region of the overlap points (52).

9. Textile pressure sensor according to one of the preceding claims,
**characterized in that**
the first and the second structures (30a, 30b) which are conductive at least in regions and possibly also the dielectric intermediate element (48) are composed of a single coherent knit, possibly comprising a plurality of different yarns.

10. Method for producing a textile pressure sensor for capacitively measuring a pressure distribution between an object and a surface according to one of Claims 1 to 9,
**characterized in that**
conductive regions for forming capacitor electrodes and/or connection webs and insulating regions for connecting the capacitor electrodes in an insulated manner are knitted to one another.

11. Method according to Claim 10,
**characterized in that**
the insulating regions are knitted onto the conductive regions.

12. Method according to either of Claims 10 and 11,
**characterized in that**
further insulating regions for forming compressible capacitor dielectrics are knitted at least between mutually opposite conductive regions which form capacitors, preferably knitted into the conductive regions such that they connect the said conductive regions to one another.

## Revendications

1. Capteur de pression textile destiné à la mesure capacitive d'une distribution de pression d'objets de forme quelconque, notamment des parties du corps, sur une surface, possédant une première structure (30a) conductrice au moins dans certaines zones et une deuxième structure (30b) conductrice au moins dans certaines zones, la première et la deuxième structure conductrices au moins dans certaines zones étant séparées l'une de l'autre par un élément intermédiaire (48) diélectrique compressible, et des zones conductrices (34) de la première structure (30a) formant des condensateurs avec des zones conductrices (34) opposées de la deuxième structure (30b), **caractérisé en ce que** la première et la deuxième structure (30a, 30b) conductrices au moins dans certaines zones sont réalisées sous la forme de tricots et comportent respectivement plusieurs zones conductrices (34) qui sont reliées ensemble par des zones non conductrices (32) fixées par tricotage.

2. Capteur de pression textile selon la revendication 1, **caractérisé en ce que** les zones conductrices (34) du tricot comportent plusieurs rangées de mailles voisines d'un fil conducteur ou de filaments conducteurs.

3. Capteur de pression textile selon la revendication 1 ou 2, **caractérisé en ce que** les bandes conductrices de la première structure (30a) sont disposées avec un angle donné par rapport aux bandes conductrices de la deuxième structure (30b) et les bandes conductrices forment des zones de recoupement (52) qui sont disposées en forme de matrice.

4. Capteur de pression textile selon l'une des revendications précédentes, **caractérisé en ce que** le tricot possède des bornes (44) incorporées, notamment fixées par tricotage, servant à relier les zones conductrices (34) à un dispositif d'interprétation électrique.

5. Capteur de pression textile selon l'une des revendications précédentes, **caractérisé en ce que** l'élément intermédiaire diélectrique (48) est réalisé sous la forme d'un tricot de préférence compressible avec effet élastique.

6. Capteur de pression textile selon l'une des revendications précédentes, **caractérisé en ce que** l'élément intermédiaire diélectrique (48) possède des éléments de rappel intégrés dans la zone des points de recoupement.

7. Capteur de pression textile selon l'une des revendications précédentes, notamment selon la revendication 6, **caractérisé en ce que** les éléments de rappel intégrés sont incorporés dans le tricot.

8. Capteur de pression textile selon l'une des revendications précédentes, notamment selon la revendication 7, **caractérisé en ce que** les éléments de rappel intégrés comportent plusieurs rangées de mailles d'un tricot compressible de manière réversible et sont disposés au moins dans la zone des points de recoupement (52).

9. Capteur de pression textile selon l'une des revendications précédentes, **caractérisé en ce que** la première et la deuxième structure (30a, 30b) conductrices au moins dans certaines zones et éventuellement aussi l'élément diélectrique (48) se composent d'un tricot cohérent unique, éventuellement constitué de plusieurs fils différents.

10. Procédé de fabrication d'un capteur de pression textile destiné à la mesure capacitive d'une distribution de pression entre un objet et une surface selon l'une des revendications 1 à 9, **caractérisé en ce que** des zones conductrices destinées à former des électrodes de condensateur et/ou des bandes de raccordement et des zones isolantes destinées servant à la liaison isolée des électrodes de condensateur entre elles sont tricotées.

11. Procédé selon la revendication 10, **caractérisé en ce que** les zones isolantes sont fixées par tricotage aux zones conductrices.

12. Procédé selon l'une des revendications 10 ou 11, **caractérisé en ce que** des zones isolantes supplémentaires destinées à former des diélectriques de condensateur compressibles sont tricotées au moins entre les zones conductrices mutuellement opposées formant des condensateurs, de préférence en reliant les zones conductrices ensemble en étant intégrées par tricotage dans celles-ci.
